(19) Europäisches Patentamt
European Patent Office
Office européen des brevets

(11) Publication number: 0 232 562 B1

(12) # EUROPEAN PATENT SPECIFICATION

(45) Date of publication of the patent specification: 28.11.90

(51) Int. Cl.[5]: C07C 231/06

(21) Application number: 86202259.7

(22) Date of filing: 15.12.86

(54) ## Process for the preparation of alpha-amino alpha-hydrogen carboxylic acid amides.

(30) Priority: 22.01.86 NL 8600132

(43) Date of publication of application: 19.08.87 Bulletin 87/34

(45) Publication of the grant of the patent: 28.11.90 Bulletin 90/48

(84) Designated Contracting States: AT BE CH DE ES FR GB IT LI NL SE

(56) References cited:
CHEMICAL ABSTRACTS, vol. 102, no. 13, 1st April 1985, page 671, abstract no. 113066u, Columbus, Ohio, US; & JP-A-59 161 341 (UBE INDUSTRIES, LTD) 12-09-1984

(73) Proprietor: STAMICARBON B.V., Mijnweg 1, NL-6167 AC Geleen(NL)

(72) Inventor: Boesten, Wihelmus H. J., Brountslaan 9, NL-6132 BJ Sittard(NL)
Inventor: Kamphuis, Johan, aureliushof 129h, NL-6215 SR Maastricht(NL)

EP 0 232 562 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of α-amino-α-hydrogen carboxylic acid amides having the formula:

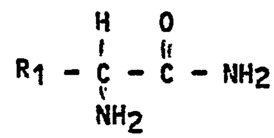

where $R_1$ represents alkyl, alkaryl, aryl, alkenyl, heteroalkyl or heteroaryl, whether or not substituted, by conversion of the corresponding amino nitriles.

The α-amino-α-hydrogen carboxylic acid amides obtained can be used in the synthesis of optically pure α-amino acids. Both the amides and the acids can be used in the pharmaceutical, agricultural and the flavours and fragrances industries.

GB-A-1548032 discloses that α-amino acid amides can be prepared by conversion of the corresponding nitriles with the aid of a ketone at a pH of 12.5-13.5. JP-A-31-44545 discloses a comparable conversion, the pH here being higher than 14. This necessitates use of a stronger base than ammonia.

The drawback of such processes resides notably in the problem that sterically hindered amino nitriles are converted only very slowly. A good example is the nitrile of tertiary leucine, with $R_1$ being t-butyl. In addition, the strongly basic alkali hydroxides and tetraalkyl ammonium hydroxides that are used necessitate removal of the inorganic salts in the purification of the α-amino acid amide obtained. This is of importance in particular if the amides obtained are to be used as substrate for enzymatic reactions, the reaction rate of an enzymatic conversion being adversely affected by high salt concentrations.

The object of the invention is to eliminate said drawbacks.

The invention therefore provides a process for the preparation of α-amino-α-hydrogen carboxylic acid amides having the formula

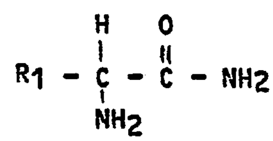

where $R_1$ represents an alkyl, alkaryl, aryl, alkenyl, heteroalkyl or heteroaryl, whether or not substituted, by conversion of the corresponding amino nitriles and is characterized in that the conversion takes place in the presence of 2–6 moles hydrogen peroxide per mole α-amino nitrile and 3–12 moles ammonia per mole α-amino nitrile.

This ensures that the conversion from nitrile to amide is effected within a few hours, independent of the bulkiness of $R_1$ • $R_1$ may, for instance, be t-butyl, 1-methylcyclohexyl and 1,1-dimethylpropyl. In addition, the amides obtained can be purified in a simple manner since the ammonia can simply be removed by distillation after decomposition of the excess hydrogen peroxide using, for instance, palladium-on-carbon.

Another advantage is that the α-amino acid amides obtained can in a simple manner be converted into the corresponding α-amino acids by means of 1 to 2 equivalents of a strong base such as NaOH or KOH. It is true that an α-amino acid can be prepared directly from the corresponding α-amino nitrile, but this requires the use of 6 equivalents of a strong acid, such as HCl. This has the disadvantage that the environment is highly corrosive in that case.

It is true that JP-A 8 331 830 discloses a conversion of an α-amino nitrile into an α-amino acid amide in which hydrogen peroxide in a basic environment is used, but this relates to the synthesis of α-methyl-α-amino acid amides rather than α-hydrogen-α-amino acid amides.

Furthermore, use is made of an alkali metal hydroxide or carbonate as base, which gives rise to the problems already described when the amide is subjected to the further treatment. In the preparation according to said publication also a very high concentration of hydrogen peroxide is used.

The process according to the invention is carried out in the presence of 2–6 moles hydrogen peroxide, in particular in the presence of about 2.2 moles hydrogen peroxide, per mole α-amino nitrile. In general it is attempted not to use more moles hydrogen peroxide per mole α-amino nitrile than is strictly necessary.

The amount of ammonia to be used in the process according to the invention may vary between 3 and 12 moles $NH_3$ per mole α-amino nitrile. In particular use is made of between 5 and 8 moles $NH_3$. Use of more than 12 moles $NH_3$ does not yield additional advantages. On the whole, the reaction proceeds well if the pH is kept above 9. Upon completion of the reaction, after decomposition of hydrogen peroxide with a reducing agent, the excess ammonia can be removed by, for instance, distillation. This way it is prevented that, besides the desired α-amino acid amides, salts are formed that subsequently have to be removed.

The process according to the invention can be carried out at various temperatures. By preference a temperature between 0 and 50°C is used, in particular one between 25 and 45°C. The amino nitriles being thermally instable, it is preferred not to use a higher temperature. The stability of the α-amino nitrile determines the allowable temperature. The α-amino nitriles decompose notable above 60°C into cyanide and ketones, upon which the cyanide may polymerize under the prevailing reaction conditions.

The process is generally carried out in solvents such as water and lower alcohols, such as methanol, ethanol, propanol, isopropanol, butanol, etc. In principle, higher alcohols may also be used, but they are less interesting because of the poorer miscibility with water. Furthermore use can be made of other organic solvents that can be mixed with water, such as dioxane and tetrahydrofuran.

When carried out as described above, the reaction is completed within a few hours.

The reaction is not affected by $R_1$, which can, therefore, in principle be chosen at random. $R_1$ will generally be an alkyl, alkenyl, aryl, alkaryl, heteroalkyl or heteroaryl group, it of course being possible for the alkyl group to be cyclic. The bulkiness of said group is not important, either, but mostly it will contain not more than 20 carbon atoms. $R_1$ may optionally be a substituted hydrocarbon residue, insofar as the substituent does not react under the reaction conditions. As such, notably compounds containing oxygen and nitrogen can be mentioned, in particular alkoxy and alkylamine.

The optically active α-amino-α-hydrogen carboxylic acid amides and the corresponding acids will generally find application in compounds consisting in part of amino acids or amino acid analogues. $R_1$ therefore preferably is an organic group, whether or not substituted, that is closely related to corresponding groups of natural amino acids. Examples are methyl, ethyl, isopropyl, 2-butyl, benzyl, hydroxymethyl, methoxymethyl, benzopyrrolyl, methoxy-substituted benzyl groups and methylimidazolyl.

The α-amino nitriles may, for instance, be synthesized by addition of hydrochloric acid to aldehydes in an ammonia-containing environment. A process for doing this is described, for instance, in Org. Synth. Collect. Vol. 3, p. 84 (R.E. Steiger).

Conversion of the nitrile into the amide may take place immediately upon addition. It is not necessary, and ofter even undesirable, to isolate the α-amino nitrile since these amino-nitriles may be thermally unstable.

After the conversion into α-amino acid amides, which are stable at the usual temperatures, first the excess hydrogen peroxide can be decomposed by means of a reducing agent, after which the ammonia can simply be evaporated. If desired, subsequently separation of the optical isomers can take place. The L-α-amino acid can be obtained by optical separation of the D,L compound, for instance by use of an amidase-containing enzyme preparation obtained from a Pseudomonasputida culture.

The optical separation of the D,L compounds could, however, also be effected using physicochemical methods, in which the amide, which already is virtually pure, can very simply be used.

The α-amino-α-hydrogen carboxylic acid amides and corresponding acids may serve as intermediaries in the synthesis of products for the agricultural, pharmaceutical and flavours and fragrances industries. Possible applications are, for instance, ampicillin, penicillin, aspartame.

The invention will be elucidated with reference to the following examples, without being restricted thereto.

## Example I

An amount of 30 ml concentrated acetic acid was slowly added to 300 ml concentrated ammonia and 70 ml NaCN (30 % solution). The temperature was kept below 40°C. At a temperature ≤ 40°C, 0.5 mole (36 g) isobutyraldehyde was dropwise added. The mixture was stirred for 3 hours at 40°C, upon which it could, if desired, without further treatment, be used for the reaction according to the invention. The amino nitrile obtained, in this case valinonitrile, can, however, also be isolated by extraction with 50 ml toluene. This obviates the need to remove the inorganic salts, present during this preparation, at the end of the next step.

## Example II

In one hour, 49 g (0.5 mole) valinonitrile, obtained in accordance with Example I, and 130 ml hydrogen peroxide (30 % = 1.1 mole) were simultaneously added dropwise to 250 ml concentrated ammonia (12 N). The temperature of the reaction mixture was kept at 30°C. After 2 hours' stirring, 1 g palladium-on-carbon was added to decompose the excess hydrogen peroxide. The palladium-on-carbon was subsequently filtered off and the aqueous layer was evaporated at reduced pressure (T ≤ 40°C). An amount of 32 g valineamide (50 %) was isolated. This yield relates to the result of both the nitrile preparation and the amide preparation according to the invention.

Example III

Tertiary leucinonitrile and hydrogen peroxide (30 %) were simultaneously added to 300 ml concentrated ammonia in amounts of 0.5 mole (56 g) and 130 ml, respectively. The tertiary leucinonitrile had been obtained from 0.5 mole pivaldehyde in a reaction that was analo gous to that of Example I. Addition of the reagents took about an hour, the temperature being kept below 40°C. After another hours's stirring, the hydrogen peroxide was reduced with 1 g 5 % palladium-on-carbon and the solid matter was filtered off. The aqueous solution was evaporated at reduced pressure (T ≤ 40°C) and 39 g tertiary leucineamide (60 %) could be recovered as white crystalline material.

Examples IV through VII

In ways analogous to Examples I and II, α-amino-α-hydrogen carboxylic acid amides were prepared from the aldehydes shown in Table 1. The results obtained when the amino nitriles were added as such and when they were added as a solution in toluene or methanol (50 %) were similar. In some cases it is possible to add an α-amino nitrile to a mixture of ammonia and hydrogen peroxide. The yields are based on two steps, from aldehyde via the nitrile to amide.

TABLE I

| example | starting material | α-amino acid amides<br>R1 | yield<br>% |
|---|---|---|---|
| IV | benzaldehyde | phenylglycineamide<br>$C_6H_5-$ | 50 |
| V | phenylacetaldehyde | phenylalanineamide<br>$C_6H_5CH_2-$ | 70 |
| VI | phenylpropionaldehyde | homophenylalanineamide<br>$C_6H_5-CH_2-CH_2-$ | 65 |
| VII | paramethoxybenzaldehyde | p-methoxyphenylglycineamide<br>$p-CH_3-O-C_6H_5-$ | 60 |

## Claims

1. Process for the preparation of α-amino-α-hydrogen carboxylic acid amides of the formula

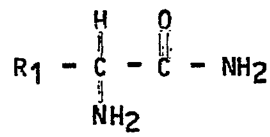

where $R_1$ represents alkyl, alkaryl, aryl, alkenyl, heteroalkyl or heteroaryl, whether or not substituted, by conversion of the corresponding amino nitriles, characterized in that the conversion takes place in the presence of 2–6 moles hydrogen peroxide per mole α-amino nitrile and 3–12 moles ammonia per mole α-amino nitrile.

2. Process according to claim 1, characterized in that use is made of about 2.2 moles hydrogen peroxide per mole α-amino nitrile.

3. Process according to claim 1 or 2, characterized in that it is carried out using 5–8 moles ammonia per mole α-amino nitrile.

4. Process according to any of claims 1–3, characterized in that it is carried out at a temperature between 0 and 50°C.

5. Process according to claim 4, characterized in that it is carried out at a temperature between 25 and 45°C.

## Patentansprüche

1. Verfahren zur Herstellung von α-Amino-α-hydrogencarbonsäureamiden der Formel

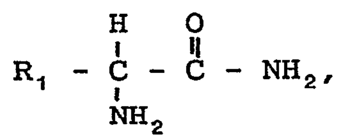

worin $R_1$ Alkyl, Alkaryl, Aryl, Alkenyl, Heteroalkyl oder Heteroaryl, egal ob substituiert oder nicht, bedeutet, durch Reaktion der entsprechenden Aminonitrile, dadurch gekennzeichnet, daß die Reaktion in Anwesenheit von 2 bis 6 Molen Wasserstoffperoxid pro Mol α-Aminonitril und 3 bis 12 Molen Wasserstoffperoxid pro Mol α-Aminonitril durchgeführt wird.

2. Verfahren nach Anspruch 1, dadurch gekennzeichnet, daß etwa 2,2 Mole Wasserstoffperoxid pro Mol α-Aminonitril verwendet werden.

3. Verfahren nach Anspruch 1 oder 2, dadurch gekennzeichnet, daß es unter Verwendung von 5 bis 8 Molen Ammoniak pro Mol α-Aminonitril durchgeführt wird.

4. Verfahren nach einem der Ansprüche 1 bis 3, dadurch gekennzeichnet, daß es bei einer Temperatur von 0 bis 50°C durchgeführt wird.

5. Verfahren nach Anspruch 4, dadurch gekennzeichnet, daß es bei einer Temperatur von 25 bis 45°C durchgeführt wird.

## Revendications

1. Procédé de préparation d'amides d'acides alpha-amino-alpha-hydrogéno-carboxyliques de formule:

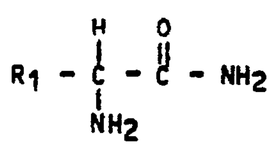

dans laquelle $R_1$ représente un radical alkyle, alcaryle, aryle, alcényle, hétéroalkyle ou hétéroaryle, substitué ou non, par conversion des amino-nitriles correspondants, caractérisé en ce que la conversion a lieu en présence de 2 à 6 moles de peroxyde d'hydrogène par mole d'alpha-amino-nitrile et 3 à 12 moles d'ammoniac par mole d'alpha-amino-nitrile.

2. Procédé selon la revendication 1, caractérisé en ce qu'on utilise environ 2,2 moles de peroxyde d'hydrogène par mole d'alpha-amino-nitrile.

3. Procédé selon la revendication 1 ou 2, caractérisé en ce qu'on effectue en utilisant 5 à 8 moles d'ammoniac par mole d'alpha-amino-nitrile.

4. Procédé selon l'une quelconque des revendications 1 à 3, caractérisé en ce qu'on l'effectue à une température comprise entre 0 et 50°C.

5. Procédé selon la revendication 4, caractérisé en ce qu'on l'effectue à une température comprise entre 25 et 45°C.